# EUROPEAN PATENT SPECIFICATION

(11) **EP 2 951 302 B1**
(45) Date of publication and mention of the grant of the patent: **02.01.2019**
(21) Application number: 14746131.3
(22) Date of filing: 04.02.2014
(51) Int. Cl.: C07K 14/725, C12N 15/00, C07H 21/04

(54) **METHODS AND COMPOSITIONS FOR TREATING GASTROINTESTINAL STROMAL TUMOR(GIST)**
VERFAHREN UND ZUSAMMENSETZUNGEN ZUR HEILUNG VON GASTROINTESTINALEN STROMATUMOREN (GIST)
MÉTHODES ET COMPOSITIONS POUR LE TRAITEMENT D'UNE TUMEUR STROMALE GASTRO-INTESTINALE (GIST)

(30) Priority: 04.02.2013 US 201361760464 P
(43) Date of publication of application: 09.12.2015
(73) Proprietor: Roger Williams Medical Center, Providence, RI 02908 (US)
(72) Inventor: KATZ, Steven, C., Providence, RI 02906 (US); JUNGHANS, Richard, P., Boston, MA 02116 (US); BAIS, Antony, Providence, Rl 02908 (US)
(74) Representative: Reitstötter Kinzebach
(86) International application number: PCT/US2014/014635
(87) International publication number: WO 2014/121264

(56) References cited:
- WO-A2-2008/045437
- US-A1- 2012 253 017
- US-A1- 2013 011 406
- P. C.R. EMTAGE ET AL: "Second-Generation Anti-Carcinoembryonic Antigen Designer T Cells Resist Activation-Induced Cell Death, Proliferate on Tumor Contact, Secrete Cytokines, and Exhibit Superior Antitumor Activity In vivo: A Preclinical Evaluation", CLINICAL CANCER RESEARCH, vol. 14, no. 24, 15 December 2008 (2008-12-15), pages 8112-8122, XP055271919, US ISSN: 1078-0432, DOI: 10.1158/1078-0432.CCR-07-4910
- SADELAIN M ET AL: "The promise and potential pitfalls of chimeric antigen receptors", CURRENT OPINION IN IMMUNOLOGY, ELSEVIER, OXFORD, GB, vol. 21, no. 2, 1 April 2009 (2009-04-01), pages 215-223, XP026058399, ISSN: 0952-7915, DOI: 10.1016/J.COI.2009.02.009 [retrieved on 2009-03-25]
- Patrizia Dentelli ET AL: "'C-KIT, by interacting with the membrane-bound ligand, recruits endothelial progenitor cells to inflamed endothelium.'", BLOOD, 15 May 2007 (2007-05-15), pages 4264-4271, XP055271924, DOI: 10.1182/blood-2006-06- Retrieved from the Internet: URL:http://www.bloodjournal.org/content/bl oodjournal/109/10/4264.full.pdf [retrieved on 2016-05-11]
- CHEN-GUANG BAI: "Stem cell factor-mediated wild-type KIT receptor activation is critical for gastrointestinal stromal tumor cell growth", WORLD JOURNAL OF GASTROENTEROLOGY, vol. 18, no. 23, 1 January 2012 (2012-01-01), page 2929, XP055274544, CN ISSN: 1007-9327, DOI: 10.3748/wjg.v18.i23.2929
- NAKAZAWA YOZO ET AL: "Anti-proliferative effects of T cells expressing a ligand-based chimeric antigen receptor against CD116 on CD34(+) cells of juvenile myelomonocytic leukemia.", JOURNAL OF HEMATOLOGY & ONCOLOGY, vol. 9, 27, 16 March 2016 (2016-03-16), pages 1-11, XP002758093, ISSN: 1756-8722, DOI: 10.1186/s13045-016-0256-3
- EMTAGE ET AL.: 'Second-generation anti-carcinoembryonic antigen designer T cells resist activation-induced cell death, proliferate on tumor contact, secrete cytokines, and exhibit superior antitumor activity in vivo: a preclinical evaluation.' CLIN CANCER RES. vol. 14, no. 24, 2008, pages 8112 - 8122, XP055271919
- DENTELLI ET AL.: 'C-KIT, by interacting with the membrane-bound ligand, recruits endothelial progenitor cells to inflamed endothelium.' BLOOD vol. 109, no. 10, 2007, pages 4264 - 71, XP055271924
- DATABASE UNIPROTKB/ SWISS -PROT [Online] 09 January 2013 'RecName: Full=T- cell surface glycoprotein CD 3 zeta chain; AltName: Full=T- cell receptor T3 zeta chain; AltName: CD _antigen= CD 247; Flags: Precursor.', XP055271975 Retrieved from NCBI Database accession no. P20963
- KATZ ET AL.: 'Open Access This content is freely available online to anyone, anywhere at any time. Anti-KIT designer T cells for the treatment of gastrointestinal stromal tumor.' J TRANSL MED. vol. 11, 21 February 2013, page 46, XP021142089
- BAI ET AL.: 'Stem cell factor-mediated wild-type KIT receptor activation is critical for gastrointestinal stromal tumor cell growth.' WORLD J GASTROENTEROL. vol. 18, no. 23, 2012, pages 2929 - 37, XP055274544
- KATO ET AL.: 'c-Kit-targeting immunotherapy for hereditary melanoma in a mouse model.' CANCER RES. vol. 64, no. 3, 2004, pages 801 - 6, XP055274547
- SHIRASU ET AL.: 'Molecular characterization of a fully human chimeric T- cell antigen receptor for tumor-associated antigen EpCAM.' J BIOMED BIOTECHNOL. 2012, page 853879, XP055274551

## Description

### CROSS-REFERENCE TO RELATED APPLICATION

This application claims benefit of priority to U.S. Provisional Application No. 61/760,464, filed February 4, 2013.

### BACKGROUND OF THE INVENTION

Gastrointestinal stromal tumor (GIST) is the most common GI mesenchymal neoplasm. Imatinib mesylate has been demonstrated to significantly prolong disease-free survival in the adjuvant setting and for patients with disseminated GIST. Unfortunately, the majority of patients with metastatic GIST who are treated with imatinib develop resistance and subsequently progressive disease. Therapeutic options are limited for patients who develop advanced GIST unresponsive to tyrosine kinase inhibitor (TKI) therapies such as imatinib. Consequently, there exists a need in the art for alternative therapies for treating GIST, particularly forms that are resistant to conventional therapies.

Bai et al. (World J Gasteroenterology, 2012, 18(23):2929-2937) report that stem cell factor-mediated wildt-type KIT receptor activation is critical for GIST cell growth. Dentelli et al. (Blood, 2007, 109(10):4264-4271) report that c-Kit recruits endothelial progenitor cells to inflamed endothelium by interaction with the membrane-bound ligand.

### SUMMARY OF THE INVENTION

The invention features a nucleic acid construct for use in a method of treating gastrointestinal stromal tumor (GIST) wherein said GIST is resistant to imatinib mesylate, wherein the nucleic acid construct encodes a chimeric immune receptor (CIR) protein including: a) an extracellular domain of KIT-ligand (KL), or a fragment thereof, wherein said KIT ligand comprises an amino acid sequence having at least 95% identity to SEQ ID NO:1, b) a transmembrane domain, and c) a cytoplasmic domain, wherein the transmembrane domain includes a transmembrane domain of the CD3 zeta chain, and the cytoplasmic domain includes a cytoplasmic domain of the CD3 zeta chain, and wherein a cell expressing said CIR protein can lyse KIT+ tumor cells *in vitro.* The invention also features a nucleic acid construct for use in a method of treating gastrointestinal stromal tumor (GIST) wherein said GIST is resistant to imatinib mesylate, wherein the nucleic acid construct encodes a chimeric CIR protein including: a) an extracellular domain of KIT-ligand, or a fragment thereof, wherein said KIT ligand comprises an amino acid sequence having at least 95% identity to SEQ ID NO:1, b) a transmembrane domain, and c) a cytoplasmic domain, wherein the transmembrane domain includes a transmembrane domain of CD28, or a fragment thereof, and the cytoplasmic domain includes a cytoplasmic domain of the CD3 zeta chain and a cytoplasmic domain of CD28, and wherein a cell expressing said CIR protein can lyse KIT+ tumor cells *in vitro.*

In certain embodiments, the extracellular domain further includes a domain of CD28, or a fragment thereof. In one embodiment, the CIR protein, when expressed in a T cell, is capable of activating the T cell in the presence of a tyrosine-protein kinase KIT positive (KIT+) tumor cell. In another embodiment the extracellular domain of the CIR protein is capable of interacting with KIT on the surface of a tumor cell when expressed in a T cell.

The invention also features a vector including the nucleic acid constructs described above and a host cell including the nucleic acid constructs and vector for use in a method of treating gastrointestinal stromal tumor (GIST) wherein said GIST is resistant to imatinib mesylate. In one aspect, the host cell is selected from the group consisting of: a T cell, a hematopoietic stem cell, a natural killer cell, a natural killer T cell, a B cell, and a cell of monocytic lineage. In certain aspects, the host cell is autologous to the subject. In other aspects, the host cell is not autologous to the subject.

The disclosure features a method of destroying a KIT+ cell, the method including administering a composition including the nucleic acid construct described above. The method can include contacting the KIT+ cell with a composition including the host cell as described above. The invention also features a nucleic acid construct or a host cell described above for use in a method of treating a subject with a KIT+ associated disease, i.e. gastrointestinal stromal tumor (GIST) wherein said GIST is resistant to imatinib mesylate .

The treatment of a subject with a KIT+ associated disease or destroying a KIT+ cell can include the administration of a second agent. In certain embodiments, the second agent is a tyrosine-kinase inhibitor. In preferred embodiments, the tyrosine-kinase inhibitor is imatinib.

In all embodiments of the invention, the KIT+ associated disease is characterized by the presence of KIT+ tumor cells. Disclosed herein are KIT+ associated diseases selected from the group consisting of: gastrointestinal stromal tumor, acute myelogenous leukemia, small-cell lung carcinoma, ovarian carcinoma, breast carcinoma, melanoma, neuroblastoma, and soft-tissue sarcomas of neuroectodermal origin. According to the invention, the KIT+ associated disease is gastrointestinal stromal tumor (GIST) wherein the GIST is resistant to imatinib mesylate.

### Definitions

By "capable of interacting with KIT" is meant an extracellular domain which recognizes and binds tyrosine-protein kinase KIT, but does not substantially recognize and bind other molecules in a sample, e.g., a human blood sample.

By "treating" is meant ameliorating a condition or symptom(s) of a condition (e.g., the symptoms of gastrointestinal stromal tumor, acute myelogenous leukemia, small-cell lung carcinoma, ovarian carcinoma, breast carcinoma, melanoma, neuroblastoma, and soft-tissue sarcomas of neuroectodermal origin). To "treat a KIT+ associated disease" (e.g., gastrointestinal stromal tumor, acute myelogenous leukemia, small-cell lung carcinoma, ovarian carcinoma, breast carcinoma, melanoma, neuroblastoma, myelodysplastic syndrome (MDS), myeloproliferative disease (MPD), aggressive systemic mastocytosis (ASM), hypereosinophilic syndrome (HES), dermatofibrosarcoma protuberans (DFSP), soft-tissue sarcomas of neuroectodermal origin, heptocullular carcinoma, and all neoplasms derived from KIT+ stem cells) refers to administering a treatment to a subject with a KIT+ associated disease to improve the subject's condition. As compared with an equivalent untreated control, such amelioration or degree of treatment is at least 5%, 10%, 20%, 30%, 40%, 50%, 60%, 70%, 80%, 90%, 95%, 99%, or 100% improvement of the diseased-state.

By "vector" is meant a DNA molecule, usually derived from a plasmid or bacteriophage, into which fragments of DNA may be inserted or cloned. A recombinant vector will contain one or more unique restriction sites, and may be capable of autonomous replication in a defined host or vehicle organism such that the cloned sequence is reproducible. A vector contains a promoter operably-linked to a gene or coding region such that, upon transfection into a recipient cell, an RNA, or an encoded protein is expressed.

By "host cell" is meant a cell into which one or more nucleic acid constructs is introduced. Host cells can be isolated from a subject and/or isolated from an outside donor. Examples of host cell include but are not limited to T cells (e.g., isolated from human peripheral blood mononuclear cell, bone marrow, and/or thymus), hematopoietic stem cells, natural killer cells, natural killer T cells, B cells, and cells of monocytic lineage (e.g., blood monocytes and macrophages).

By "chimeric immunoreceptor" or "CIR" is meant a fusion protein which, when expressed in a host cell, contains an extracellular domain that specifically binds to a target protein and a cytoplasmic domain that modulates activation of the host cell.

By "KIT-ligand" is meant a cytokine that binds to tyrosine-protein kinase KIT (KIT), c-KIT receptor, or CD117. KIT-ligand binding to KIT can cause KIT to form a dimer that activates the intrinsic tyrosine kinase activity of the protein. KIT-ligand is also known in the art as steel factor or stem cell factor (SCF). "KIT-ligand" includes a polypeptide having at least residues corresponding to 1-273 of human KIT-ligand (SEQ ID NO:1) or e.g., a sequence having substantial identity to that of the extracellular domain of cKIT ligand (e.g., encoded by SEQ ID NOS:2,3 (italicized nucleotides indicate Ncol and BamHI restriction sites, respectively)).
**SEQ ID NO:1 Human KIT-ligand amino acid sequence**

| | | |
|---|---|---|
| LOCUS | AAI26167 273 aa | linear PRI 23-OCT-2006 |
| DEFINITION | KIT ligand [Homo sapiens]. | |
| ACCESSION | AAI26167 | |
| VERSION | AAI26167.1 GI:116496627 | |
| DBSOURCE | accession BC126166.1 | |
| 1  | | |
| (5'-gattccaggaattgatttcc*ccatgg*caaagaagacacaaacttg-3' **SEQ ID NO:2** 5'-ctaagctctagccaattgaatt*ggatcc*gtgtaggctggagtctcc-3' **SEQ ID NO:3)** | | |

By "CD28 cytoplasmic domain" is meant a polypeptide having the C-terminal region of CD28 that is located in the cytoplasm when expressed in a T cell, e.g., a polypeptide having the amino acid sequence of amino acids 127-234 of SEQ ID NO:4. The term "CD28 cytoplasmic domain" is meant to include any CD28 fragment that maintains the ability to modulate activation of T cells and is substantially identical to amino acids 127-234 of SEQ ID NO:4 over the length of the polypeptide fragment.
**SEQ ID NO:4 Human CD28 amino acid sequence**

LOCUS       NP_006130                220 aa            linear   PRI 11-APR-2010
DEFINITION  T cell-specific surface glycoprotein CD28 precursor [Homo sapiens].
ACCESSION   NP_006130
VERSION     NP_006130.1  GI:5453611
DBSOURCE    REFSEQ: accession NM_006139.2

      1 MLRLLLALNL FPSIQVTGNK ILVKQSPMLV AYDNAVNLSC KYSYNLFSRE FRASLHKGLD
     61 SAVEVCVVYG NYSQQLQVYS KTGFNCDGKL GNESVTFYLQ NLYVNQTDIY FCKIEVMYPP
     21 PYLDNEKSNG TIIHVKGKHL CPSPLFPGPS KPFWVLVVVG GVLACYSLLV TVAFIIFWVR
    181 SKRSRLLHSD YMNMTPRRPG PTRKHYQPYA PPRDFAAYRS

By "CD3 zeta" is meant a polypeptide having polypeptide having the amino acid sequence of SEQ ID NO:5. The term "CD3 zeta" also includes polypeptide fragments that maintain the ability to modulate activation of T cells and is substantially identical to SEQ ID NO:5 over the length of the protein fragment.

### SEQ ID NO:5 Human CD3 zeta amino acid sequence

LOCUS       NP_000725                163 aa            linear   PRI 11-APR-2010
DEFINITION  T cell receptor zeta chain isoform 2 precursor [Homo sapiens].
ACCESSION   NP_000725
VERSION     NP_000725.1  GI:4557431
DBSOURCE    REFSEQ: accession NM_000734.3

        1 MKWKALFTAA ILQAQLPITE AQSFGLLDPK LCYLLDGILF IYGVILTALF LRVKFSRSAD
       61 APAYQQGQNQ LYNELNLGRR EEYDVLDKRR GRDPEMGGKP RRKNPQEGLY NELQKDKMAE
      121 AYSEIGMKGE RRRGKGHDGL YQGLSTATKD TYDALHMQAL PPR

By "activating a T cell" is meant inducing a T cell expressing the CIR of the invention to release interleukin 2 (IL-2) which acts upon the T cell in an autocrine fashion. The activated T cells also produce the alpha sub-unit of the IL-2 receptor (CD25 or IL-2R), enabling a fully functional receptor that can bind with IL-2, which in turn activates the T cell's proliferation pathways.

By the term "tumor cell" is meant a component of a cell population characterized by inappropriate accumulation in a tissue. This inappropriate accumulation may be the result of a genetic or epigenetic variation that occurs in one or more cells of the cell population. This genetic or epigenetic variation causes the cells of the cell population to grow faster, die slower, or differentiate slower than the surrounding, normal tissue. The term "tumor cell" as used herein also encompasses cells that support the growth or survival of a malignant cell. Such supporting cells may include fibroblasts, vascular or lymphatic endothelial cells, inflammatory cells or co-expanded non-neoplastic cells that favor the growth or survival of the malignant cell. The term "tumor cell" is meant to include cancers of hematopoietic, epithelial, endothelial, or solid tissue origin. The term "tumor cell" is also meant to include cancer stem cells

By "KIT+ tumor cell" is meant a cell expressing KIT associated with a tumor.

By "KIT+ associated disease" is meant a disease that is characterized by increased KIT expression or aberrant KIT activity (e.g., KIT activation) in a variety of cell types, including but not limited to: mast cells, hematopoietic progenitor cells, melanocytes, germ cells, and/or gastrointestinal pacemaker cells. A KIT+ associated disease can arise from KIT+ stem cells. Examples of KIT+ associated diseases include but are not limited to: gastrointestinal stromal tumor, acute myelogenous leukemia, small-cell lung carcinoma, ovarian carcinoma, breast carcinoma, melanoma, neuroblastoma, myelodysplastic syndrome (MDS), myeloproliferative disease (MPD), aggressive systemic mastocytosis (ASM), hypereosinophilic syndrome (HES), dermatofibrosarcoma protuberans (DFSP), soft-tissue sarcomas of neuroectodermal origin, heptocullular carcinoma, and all neoplasms derived from KIT+ stem cells.

By "substantially identical" is meant a nucleic acid or amino acid sequence that, when optimally aligned, for example using the methods described below, share at least 60%, 65%, 70%, 75%, 80%, 85%, 90%, 95%, 96%, 97%, 98%, 99%, or 100% sequence identity with a second nucleic acid or amino acid sequence. Percent identity between two polypeptides or nucleic acid sequences is determined in various ways that are within the skill in the art, for instance, using publicly available computer software such as Smith Waterman Alignment (Smith, T. F. and M. S. Waterman (1981) J Mol Biol 147:195-7); "BestFit" (Smith and Waterman, Advances in Applied Mathematics, 482-489 (1981)) as incorporated into GeneMatcher Plus™, Schwarz and Dayhof (1979) Atlas of Protein Sequence and Structure, Dayhof, M.O., Ed pp 353-358; BLAST program (Basic Local Alignment Search Tool; (Altschul, S. F., W. Gish, et al. (1990) J Mol Biol 215: 403-10), BLAST-2, BLAST-P, BLAST-N, BLAST-X, WU-BLAST-2, ALIGN, ALIGN-2, CLUSTAL, or Megalign (DNASTAR) software. In addition, those skilled in the art can determine appropriate parameters for measuring alignment, including any algorithms needed to achieve maximal alignment over the full length of the sequences being compared. In general, for proteins or nucleic acids, the length of comparison can be any length, up to and including full length (e.g., 5%, 10%, 20%, 30%, 40%, 50%, 60%, 70%, 80%, 90%, 95%, or 100%). Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

By "subject" is meant a mammal (e.g., a human or non-human).

Other features and advantages of the invention will be apparent from the following Detailed Description, the drawings, and the claims.

### BRIEF DESCRIPTION OF THE DRAWINGS

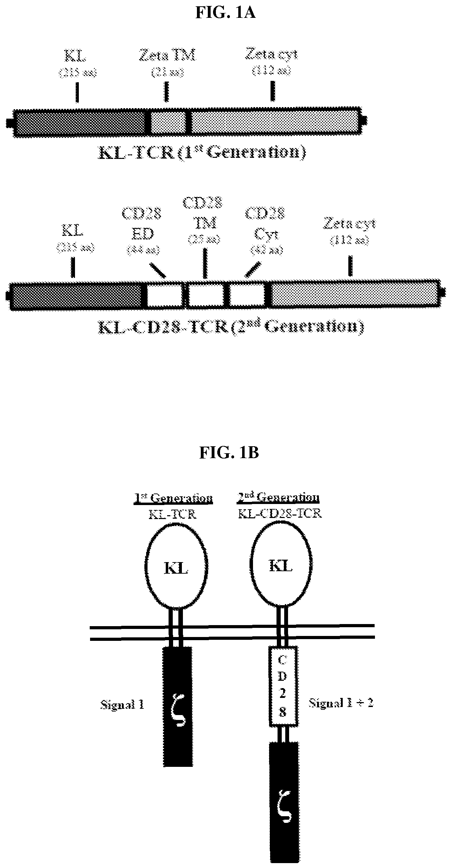

**Figures 1A-1B** are depictions of the structure of anti-KIT chimeric immune receptor. Figure 1A shows a schematic diagram of 1^{st} and 2^{nd} generation CIR genetic constructs. Figure 1B shows the structure of 1^{st} and 2^{nd} generation CIR. Anti-KIT CIRs were re-engineered from anti-CEA retroviral vector constructs, whereby the extracellular domain of KIT ligand (KL) was amplified and cloned to replace the anti-CEA extracellular domain.

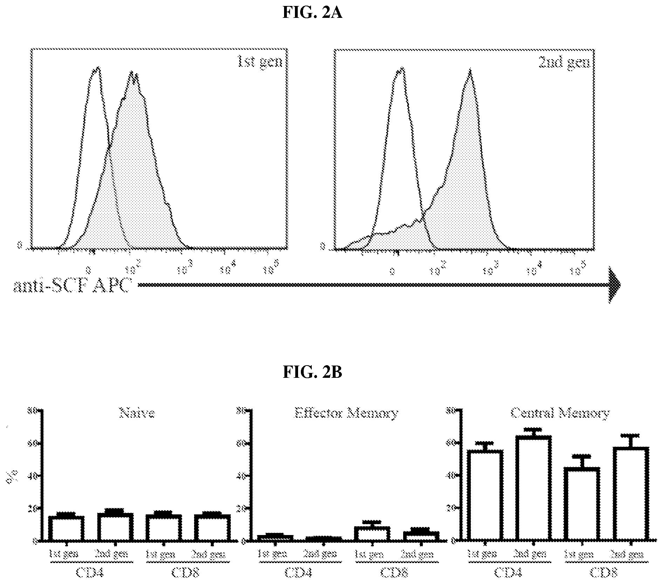

**Figures 2A-2B** are graphs showing the transduction efficiency and phenotype of human anti-KIT designer T cells. Figure 2A shows a graph of isolated PBMC and primary human T cells activated and transduced with retrovirus expressing KIT-specific CIRs. Shaded histograms represent designer T cells and open curves represent untransduced cells. Figure 2B shows flow cytometric analysis of the phenotype of 1^{st} and 2^{nd} generation transduced designer T cells demonstrating that T cells of a central memory phenotype (CD45RO+CD62LCCR7+) were in the majority. Data are representative of three or more repetitions.

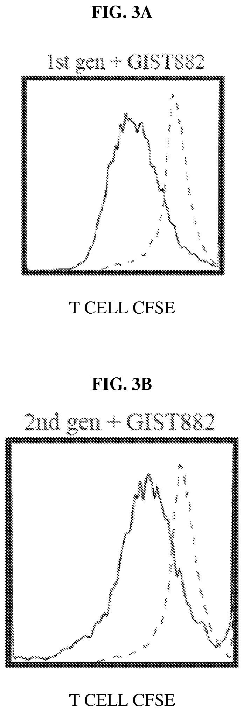

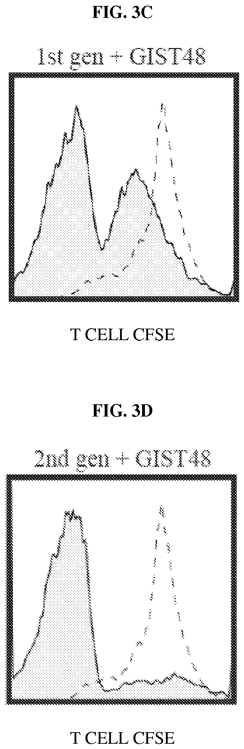

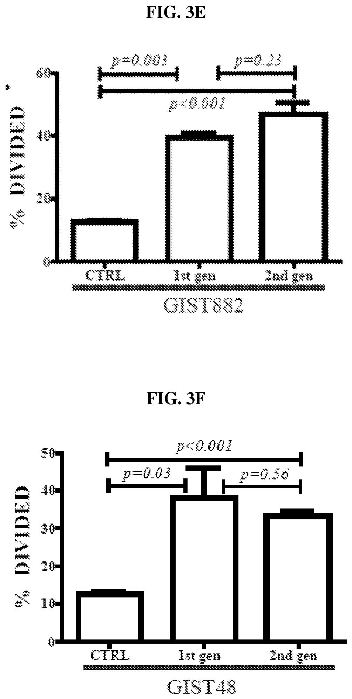

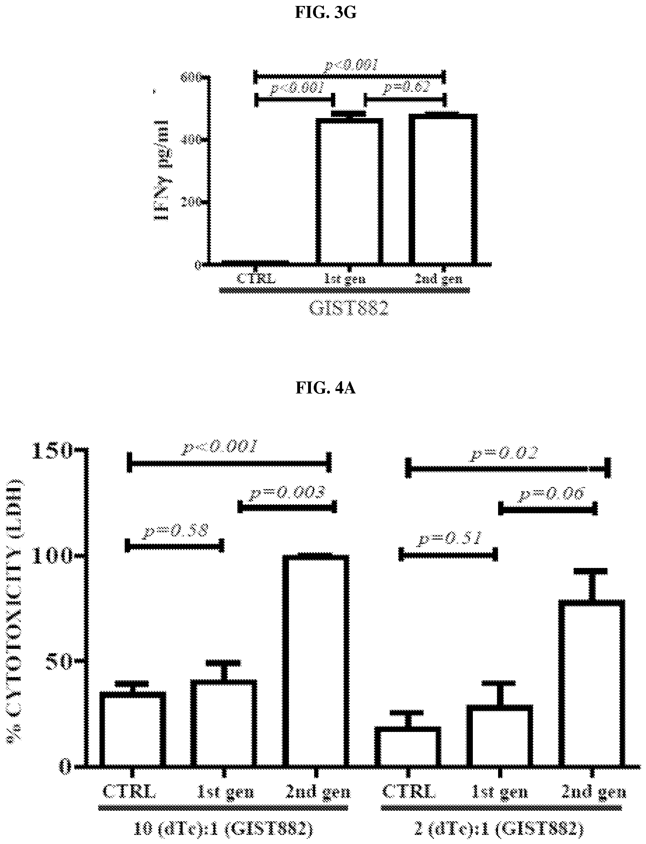

**Figures 3A-3G** are graphs showing that human anti-KIT designer T cells retain proliferative ability in vitro. 1^{st} and 2^{nd} gen designer T cells were co-cultured with human KIT+ GIST cell lines, GIST 882 in Figures 3A-3B, and 3E and GIST 48 in Figures 3C-3D, and 3F. Designer T cells and untransduced CTRL T cells were stained with CFSE and proliferation assessed by gating on CD3+ cells to measure CFSE dilution. To confirm designer T cells activated by KIT+ GIST cell lines, IFNγ production was measured by ELISA, and was found to be in the range of 462-475 pg/mL, while that of unmodified T cells (CTRL) was negligible (G). Data are representative of three or more repetitions.

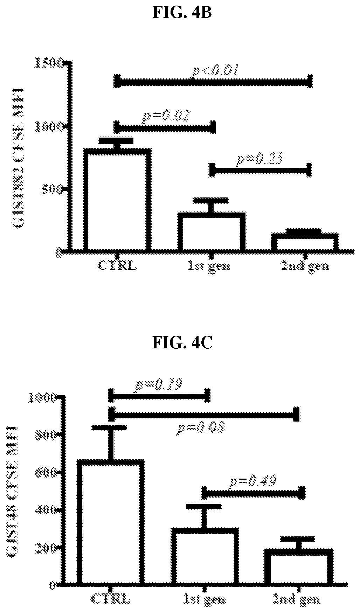

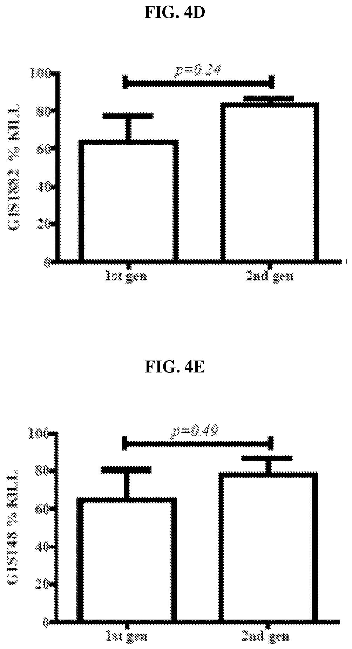

**Figures 4A-4E** are graphs showing the ability of human anti-KIT designer T cells to effectively lyse KIT+ tumor cells. Figure 4A shows results from a LDH assay to evaluate enzymatic release following tumor cell lysis performed on supernatant from co-culturing of 1^{st} and 2^{nd} generation designer T cells with irradiated GIST 882 cells. Maximal release was defined by the highest experiment values. Figures 4B-4C show graphs of irradiated GIST-88s or GIST 48 cells labeled with CFSE and cultured with unlabelled T cells to confirm the cytotoxic ability of designer T cells. Tumor cell death was quantified by measuring the decrease in CFSE fluorescence by gating on remaining live cells. Figures 4D-4E show quantified results of the percent kill of 1^{st} and 2^{nd} gen designer T cells by normalizing MFI data to that of the unmodified cells (CTRL). Data are representative of at least three repetitions.

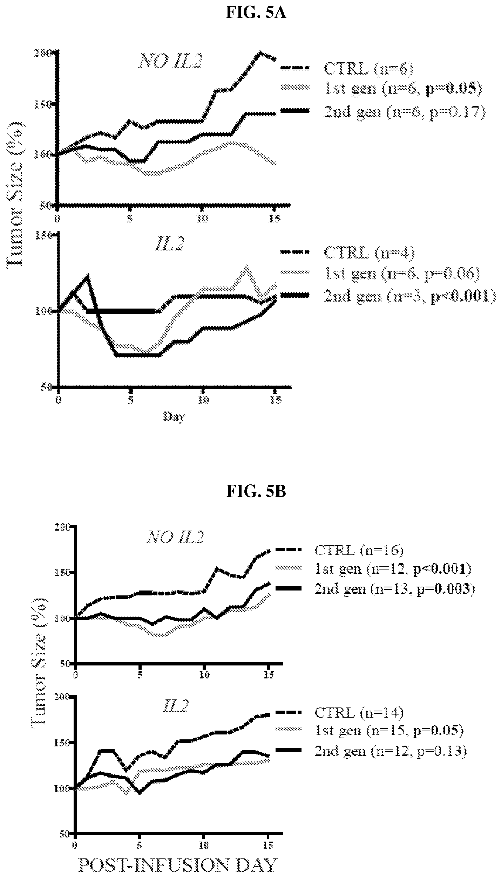

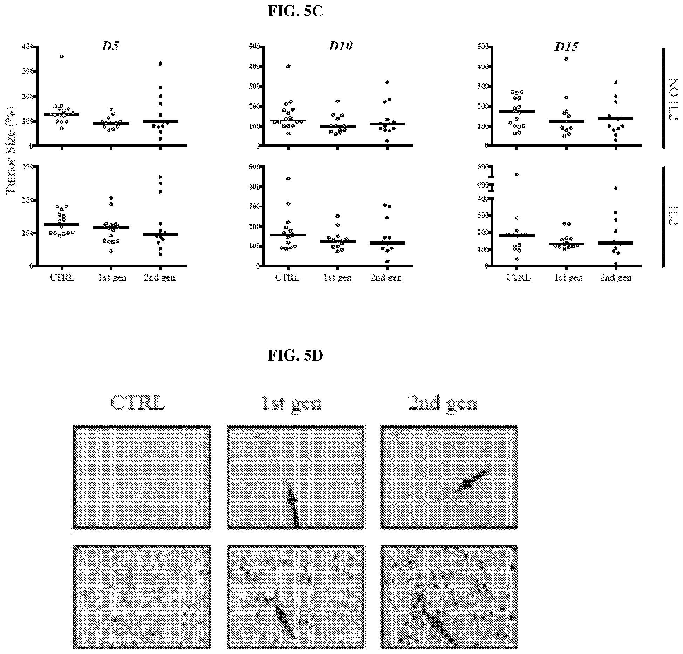

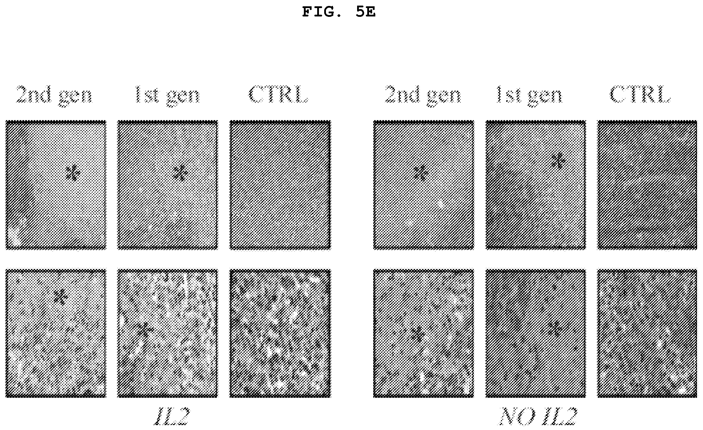

**Figures 5A-5E** are results of in vivo testing of human anti-KIT designer T cells. Figure 5A show median results from a single representative's experiment of subcutaneous xenograft model whereby GIST 882 cells were injected subcutaneously into immunodeficient mice prior to treatment with human anti-KIT designer T cells and Figure 5B shows results from pooled data from 3 experiments. Figure 5C shows plots of the median tumor sizes at Day 5, Day 10, and Day 15 post-infusion indicated by horizontal bar along with individual tumor measurements represented by individual data points. Figure 5D shows immunohistochemistry blots (top row 10x and bottom row 40x) detecting tumor infiltrating CD3+ T cells (arrows). Figure 5E shows routine histology blots used to assess the degree of tumor necrosis (asterisks) in mice treated with 1^{st} or 2^{nd} gen designer T cells, both with and without supplemental IL2 (left column 10x and right column 40x).

### DETAILED DESCRIPTION OF THE INVENTION

In general, the invention features nucleic acid constructs for use in a method of treating gastrointestinal stromal tumor (GIST) wherein said GIST is resistant to imatinib mesylate, wherein the nucleic acid constructs encode chimeric immune receptor (CIR) proteins that include KIT-ligand (KL) or stem cell factor (SCF). These nucleic acid constructs, when expressed in cells (e.g., a subject's T cells), are useful for the treatment of diseases associated with increased KIT expression or aberrant KIT activity. Examples of such diseases include gastrointestinal stromal tumors (GIST), particularly those resistant to imatinib and other conventional therapies. Certain embodiments include an engineered CIR that contains the natural ligand for KIT (e.g., KIT-ligand (KL) or stem cell factor (SCF). For example, KIT-ligand (KL) or stem cell factor (SCF) can be fused to the CD3ζ chain component of the T cell receptor (1^{st} generation, 1^{st} gen) or CD3ζ + the CD28 co-stimulatory molecule (2^{nd} generation, 2^{nd} gen). The 2^{nd} gen T cells express the construct that targets KIT+ tumors while, at the same time, integrating CD28 co-stimulatory signals. In the examples set forth below, such 1^{st} and 2^{nd} gen T cells were produced and tested in vitro and in vivo to demonstrate their efficacy in destroying KIT+ tumor cells.

### Extracellular domains

The CIR of the invention features an extracellular domain able to specifically bind tyrosine protein kinase KIT (KIT) and to direct the CIR of the invention to KIT+ expressing tumor cells. Therefore, the extracellular domain of the CIR includes, e.g., the full-length stem cell factor (SCF) or the KIT-ligand (KL), or fragments thereof. Further, CIR proteins are described herein, wherein the extracellular domain includes a binding moiety specific for inhibiting dimerization of KIT, inhibiting tyrosine kinase activity, and/or KIT phosphorylation, and/or antibody fragments against KIT (e.g., an anti-cKIT antibody for example those commercially available from Abcam, Biolegend, Genway Biotech, etc).

The extracellular domain can optionally include a further protein tag useful for purification of the expressed CIR, e.g., a c-myc tag (EQKLISEEDL) of human origin, at the N-terminus, a Z domain, HA tag, FLAG tag, and/or GST tag. The protein tag is preferably chosen such that the tag does not obstruct KL interaction with KIT. The extracellular domain can also optionally include a further protein useful for imaging, e.g., fluorescent proteins. Inclusion of these proteins can facilitate future study of the constructs and to create constructs that are more effective.

### Transmembrane domains

The CIR of the invention features transmembrane domains, e.g., those derived from CD28 or CD3 zeta. The inclusion of the transmembrane region of the zeta chain or the transmembrane and partial extracellular domain of CD28 provides the capability of intermolecular disulfide bonds. CIRs containing these transmembrane domains are predicted to form disulfide-linked dimers through a cysteine residue located in the transmembrane of zeta or in the proximal cysteine residue located in the partial extracellular domain of CD28 (position 123 of CD28), mimicking the dimer configuration of native zeta and CD28.

### Cytoplasmic domains

The CIR of the invention also feature a cytoplasmic domain for modulating activation of the host T cells when bound to KIT+ tumor cells and to activate T cell based cytotoxic responses to attack the KIT+ tumor cells. Cytoplasmic domains for use in the CIRs of the invention comprise a cytoplasmic domain CD3 zeta or both a cytoplasmic domain of CD3 zeta and a cytoplasmic domain of CD28. The disclosure also features the fusion of polypeptides derived from multiple extracellular domains for potentiating activation of T cells when bound to KIT+ tumor cells (e.g., a cytoplasmic domain that includes both active fragments of CD3 zeta and CD28 or a cytoplasmic domain that includes only CD3 zeta).

### Nucleic acid constructs

The nucleic acid constructs of the invention are useful for expressing CIR constructs in host cells (e.g., T cells isolated from a subject). CIR constructs can be included in a single nucleic acid construct or multiple nucleic acid constructs. Nucleic acid sequences encoding the CIR can be changed to codons more compatible with an expression vector or host cell used for producing the CIR. Codons may be substituted to eliminate restriction sites or to include silent restriction sites, which may aid in cloning of the nucleic acid in an expression vector and processing of the nucleic acid in the selected host cell. In order to facilitate transfection of host cells, the nucleic acid construct can be included in a viral vector (e.g., a retroviral vector or adenoviral vector) or be designed to be transfected into a host cell via electroporation or chemical means (e.g., using a lipid transfection reagent).

### Host cells

The host cells of the invention can be derived from any mammalian source containing T cells (e.g., human peripheral blood mononuclear cell, bone marrow, and/or thymus, and isolated from, e.g., a subject's own cells or from another donor source), hematopoietic stem cells, natural killer cells, natural killer T cells, B cells, and cells of monocytic lineage (e.g., blood monocytes and macrophages). The host cells are transfected or infected with the nucleic acid constructs of the invention (e.g., nucleic acid constructs encoding a CIR). Prior to administration to a patient, the host cell can be expanded in cell culture. In one embodiment, the modified host cells are administered to the patient from whom they were originally isolated. In another embodiment, the modified host cells are administered to a patient from another source from which they are isolated.

### Conditions and disorders

Histologic studies confirm that the CIR of the invention localize to KIT+ tumors and mediate tumor necrosis after intravenous infusion. In some embodiments, single or multiple infusions may be necessary to achieve complete regression of established tumors. In particular embodiments, addition of IL15 and IL21 to promote an effector phenotype may offer the potential of enhancing the efficacy of the CIR of the invention. In specific embodiments, significant delays in tumor growth in the in vivo model support the potential utility of the CIR of the invention for the treatment of GIST. In another embodiment, the CIR of the invention may also be useful for the treatment of KIT+ associated diseases. Examples of KIT+ associated diseases include but are not limited to: acute myelogenous leukemia, small-cell lung carcinoma, ovarian carcinoma, breast carcinoma, melanoma, neuroblastoma, myeloproliferative disease (MPD), aggressive systemic mastocytosis (ASM), hypereosinophilic syndrome (HES), dermatofibrosarcoma protuberans (DFSP), soft-tissue sarcomas of neuroectodermal origin, and/or any other disease characterized by the overexpression and/or hyper-activation of KIT. The claimed invention concerns the treatment of gastrointestinal stromal tumor (GIST) wherein said GIST is resistant to imatinib mesylate.

### Additional agents and combination therapy

The CIRs of the invention are useful for GIST treatment and treatment of other KIT+ associated diseases that can be used alone or in combination with other therapies, including tyrosine kinase inhibitors (TKIs) and other immunomodulatory agents and/or therapy. Imatinib has been reported to induce regulatory T cell apoptosis and its efficacy was enhanced by concurrent immunotherapy. Adding imatinib to anti-KIT designer T cells infusions may augment efficacy through favorable immunomodulation within the tumor microenvironment, allowing the CIR to mediate enhanced tumor cell lysis. Other TKIs that can be used with the CIR of the invention include, but are not limited to, erlotinib, gefitinib, lapatinib, sunitinib, sorafenib, nilotinib, bosutinib, neratinib, and vatalanib. The CIRs of the invention can also be co-administered with immunomodulatory agents such as anti-PD1 and anti-CTLA4 antibodies. In any of the treatment methods described herein, the CIRs of the invention can also be co-administered with myeloablative preconditioning as described in Dudley et al., J Clin Oncol. 26:5233-5239, 2008

### EXAMPLES

An anti-KIT CIR was constructed that can be utilized to reprogram human T cells to recognize and kill KIT+ GIST tumors. The natural ligand for KIT, SCF, confers anti-tumor specificity. Murine and human T cells were transduced with high levels of efficiency, and in vitro studies confirmed that anti-KIT designer T cells proliferated and secreted IFNγ in response to KIT+ GIST cells. The designer T cells were also able to lyse two KIT+ cell lines. Using a xenograft model, it was demonstrated that systemic infusions of 1^{st} and 2^{nd} gen anti-KIT designer T cells resulted in significant reductions in tumor growth rates. Taken together, the study of anti-KIT designer T cells supports their further development as a potential novel treatment for KIT+ neoplasms.

### Materials and methods

### Retroviral vector construction

First and second generation anti-KIT CIR were re-engineered from the anti-CEA retroviral vector expression constructs previously described in Emtage et al., Clin Cancer Res 14:8112-8122, 2008. The extracellular domain of cKIT ligand (Genebank BC069733.1, cDNA clone MGC:97379) spanning the N-terminal start codon to the transmembrane start was PCR amplified from ATTC clone 010560371 using primers incorporating Ncol (italicized nucleotides in SEQ ID NO:2) and BamHI (italicized nucleotides in SEQ ID NO:3) restriction sites and cloned in-frame to replace the anti-CEA extracellular domain.

Nucleic acid encoding extracellular domain of cKIT ligand:
(5'-gattccaggaattgatttcc*ccatgg*caaagaagacacaaacttg-3' **SEQ ID NO:2**
5'-ctaagctctagccaattgaatt*ggatcc*gtgtaggctggagtctcc-3' **SEQ ID NO:3)**

### Designer T cell production

Human peripheral blood mononuclear cells (PBMC) were obtained from random donor whole blood filtrate (Rhode Island Blood Center, Providence, RI). Blood filters were washed with sterile PBS (Cellgro, Manassas, VA) and PBMC were isolated by density gradient separation with Histopaque (Sigma-Aldrich, St. Louis, MO) according to manufacturer directions. PBMC were seeded at a density of 2 × 10⁶ cells/ml, and activated on anti-CD3 coated (OKT3, eBioscience, San Diego, CA) 750ml flasks with 2 µg/mL anti-CD28 (CD28.2, eBioscience) and 300 U/mL of human IL-2 in AIM V medium (Invitrogen, Grand Island, NY) supplemented with 5% heat inactivated sterile human serum (Valley Biomedical, Winchester, VA). 293T-HEK phoenix amphotropic cells (Orbigen, Allele Biotechnology, San Diego, CA) were transfected with 50 µg 1^{st} or 2^{nd} gen cKIT ligand CIR retroviral plasmid using LipoD283 (SignaGen Laboratories, Rockville, MD). Viral supernatant was harvested for transduction of NIH-3T3 PG13 retrovirus packaging cells (ATTC: CRL-10686) cells that had reached 80% confluence. PG13 cells were cultured at 37°C and supernatant was harvested and filtered through 0.45µm filters (Corning, Corning NY) when cells reached 80% confluence. After 24-48 hours of culture, PBMC were seeded on retronectin-coated (20 µg/mL, Takara Bio, Otsu, Shiga, Japan) wells of a 6-well plate and were transduced with anti-KIT CIR vector as described in Emtage et al., Clin Cancer Res 14:8112-8122, 2008 to create designer T cells. Cells were transduced with supernatant containing either anti-KIT CIR vector (1^{st} gen) or anti-KIT CIR vector with additional CD28 moiety (2^{nd} gen). Transduced T cells were maintained in AIM V medium supplemented with 5% heat inactivated sterile human serum and 100 IU/ml IL-2. Expression of KIT-specific CIR on designer T cells was evaluated by flow cytometric analysis of staining with Reprokine anti-SCF mAb (Fx2) conjugated to allophycocyanin-XL (Chromaprobe, Maryland Hts, MO). Cell were also stained with antibodies against human CD3 (Sk7), CD4 (RPA-T4), CD8 (SK1), CD62L, CD45RO, CD197 (CCR7, 150503), and CD25 (M-A251), which were conjugated to FITC, PE, PerCP, allophycocyanin, allophycocynanin-Cy7, or Pe-Cy7 (BD Biosciences, Franklin Lakes NJ). For FoxP3 intracellular staining, samples were fixed, permeabilized, and stained with FoxP3 conjugated to PE as per manufacturer's protocol (BD).

### Cell proliferation assay

Flow cytometry-based division assays were performed to analyze the proliferation of 1^{st} and 2^{nd} gen designer T cells in response to stimulation by KIT+ human GIST cell lines. Designer T cells were labeled with 1 µM carboxyfluorescein diacetate succinimidyl ester (CFSE, Invitrogen) and were added at a 4:1 ratio with KIT+ GIST 882 and GIST 48 cells in a 96-well round-bottom plate, with 1 x 10⁵ dTc added per well. GIST 48b, which lacks KIT surface expression, was used a negative control. Tumor cells were irradiated at 5000 rad. Co-culture was incubated for 5 days, at which point supernatant was isolated and cells were analyzed by flow cytometry. Supernatant was analyzed by cytometric bead array for IFN-γ levels (BD Biosciences). Cytokine production results were also quantified by human IFN-γ ELISA assay for confirmation (Biolegend).

### Cytotoxicity assays

1^{st} and 2^{nd} gen designer T cells were cultured with KIT+ GIST 882 or GIST 48 cells in order to evaluate their cytotoxic ability in an LDH assay (Roche, Indianapolic, IN) performed according to the manufacturer's protocol. Tumor cells were irradiated at 5000G for 50 minutes. Cytotoxic ability was evaluated for 1^{st} gen designer T cells, 2^{nd} gen designer T cells, and untransduced human T cells, which were added at various effector-to-target ratios. Cytotoxicity results from the LDH assay were further confirmed by flow cytometric analysis of tumor cell death. GIST cells were irradiated as previously described and labeled with CFSE while designer T cells were unstained. Loss of CFSE+ cells was analyzed with flow cytometry and cytotoxicity was calculated using the following formula: mean fluorescence index (MFI) 1^{st} or 2^{nd} gen designer T cells divided by MFI untransduced T cells. Tumor cells were stained with Annexin-V (BD Biosciences).

### In vivo tumor studies

Six-week-old male immunodeficient mice (NU/J) were purchased from Jackson Laboratories (Bar Harbor, ME) and experiments were conducted in compliance with the guidelines of the Roger Williams Medical Center Institutional Animal Care and Use Committee. The GIST cell lines were maintained at 37°C in IMDM with 1% I-glutamine (Invitrogen) supplemented with 15% FBS, and 1% Penicillin/Streptomycin/Amphomycin (Cellgro). Subcutaneous flank injections of 3 × 10⁷ KIT+ GIST 882 cells in 200 µl sterile PBS were administered bilaterally. Designer T cells or untransduced human T cells were injected (1 × 10⁷ in 200 µl PBS) via tail vein. For experimental groups with IL-2, Alzet 7-day micro-osmotic pumps (Durcet, Cupertino CA) were filled with IL-2 according to the manufacturer's protocol and implanted subcutaneously. Pumps were set to deliver at a rate of 10,000 IU/h (550 pg/h). Tumors were measured in two dimensions with calipers, and measurements were obtained daily from the time of T cell injection until the conclusion of the study. The average of right and left flank tumors was used for each mouse, and measurements were normalized to initial tumor size. After sacrifice, tumors were excised and sent to the University of Massachusetts, Worchester Medical Center Experimental Pathology Service Core, for histological sectioning and staining. Sections were stained for routine H&E and anti-CD3 immunohistochemistry. Slides were analyzed at the Pathology department at Roger Williams Medical Center, and photographs were taken under 10× and 40× magnification.

### Statistics

Statistics were calculated using GraphPad Prism V5.00 for Windows (GraphPad Software, San Diego, CA). Statistical significance for proliferation and cytotoxicity assays was determined using the two-tailed Student t test, and values with p≤0.05 were classified statistically significant. Tumor size median values are presented and logistic regression was used to compare growth curve slope and elevation among groups. Cell proliferation analysis with calculation of division peaks was performed using FlowJo software (Treestar, Ashland, OR).

### Example 1: Engineering of anti-KIT chimeric immune receptors and production of designer T cells

The aim of this study was to construct and test the function of KIT-specific CIR expressed by human peripheral blood T cells for pre-clinical development. The anti-KIT CIR construct was based on a pre-existing anti-CEA format described in Emtage et al., Clin Cancer Res 14:8112-8122, 2008. The anti-CEA sFv fragment was replaced with the extracellular domain of KL. 1^{st} and 2^{nd} gen constructs were prepared (Figure 1A). The 2^{nd} gen construct contains CD28 to provide co-stimulation. The KL component is expressed on the extracellular aspect of the CIR to enable interaction with KIT on the surface of target tumor cells (Figure 1B). The constructs were confirmed by direct DNA sequencing prior to transduction of activated lymphocytes.

Following activation and transduction, CIR expression was confirmed by flow cytometry with an anti-KL antibody. Retroviral Transduction of activated murine splenocytes, which was used as a preliminary assessment, resulted in 1^{st} and 2^{nd} gen CIR expression rates of 27% (range, 16-41). Following optimization of the protocol, transduction of activated human PBMC (Figure 2A) yielded mean transduction rates of 50% (range, 33-74) and 42% (range, 24-62) for 1^{st} and 2^{nd} gen human designer T cells respectively, with no significant difference between the two CIR versions (p=0.67). After stimulation of PBMC with anti-CD3, anti-CD28, and IL2, >70% of the cells were CD3+ and a central memory phenotype (CD45RO+CD62L+ CCR7+) predominated (Figure 2B). Fewer than 30% of cells had a naive (CD45RO-CD62L+) or effector memory (CD45RO+CD62L+CCR7-) phenotype, and less than 10% of transduced T cells from both generations had a regulatory T cell phenotype (CD25+FoxP3+) with no differences between the groups. For 1^{st} gen dTc, 33.4% of T cells were CD4+CD8- and 52.7% were CD8+CD4-, while the corresponding values for 2^{nd} gen dTc were 35.5% and 52.6%. The CD4:CD8 ratio did not change after transduction or exposure to KIT+ tumor. For all subsequent experiments, designer T cells were used in bulk, without fractionating by CD4 or CD8 expression, in keeping with current clinical practice.

### Example 2: Proliferation of anti-KIT designer T cells in the presence of KIT+ tumor cells

To test the proliferative capacity of human T cells expressing anti-KIT CIR, we cultured the dTc in the presence of two human KIT+ GIST cell lines, GIST882 and GIST48. In the presence of GIST882 and GIST48, dTc expressing either the 1^{st} or 2^{nd} gen anti-KIT CIR proliferated to a greater extent when compared to untransduced T cells (CTRL) as determined by CFSE dilution (Figure 3A). When cultured with GIST882, 39% of the 1^{st} gen and 47% of the 2^{nd} gen dTc divided (p<0.001 compared to CTRL), with no significant difference between the two CIR formats (p=0.23, Figure 3B). Likewise, in the presence of imatinib resistant GIST48 cells, 33-38% of the dTc divided after 3 days in culture which was significantly higher than CTRL cells (p≤0.03 compared to CTRL), with no significant difference between the two CIR formats (p=0.56, Figure 3C). The requirement of KIT+ tumor cells for dTc proliferation was confirmed by the minimal proliferation that resulted when culturing dTc in the presence of KIT-GIST 48B cells as shown, and CIR-activated T cells did not proliferate in the presence of KIT+ tumor. IFNγ production confirmed dTc activation by KIT+ tumor and was found to be in the range of 462-475 pg/ml, while production by CIR- T cells was negligible (p<0.001, Figure 3D). Co-culture of anti-KIT dTc with KIT- control tumor cells did not result in significant IFNγ production.

### Example 3: Lysis of KIT+ tumor cells by anti-KIT designer T cells

The hallmark of effective adoptive cellular immunotherapy is the ability of the product to lyse tumor cells in a specific fashion. To this end, in vitro assays were performed to determine if designer T cells expressing anti-KIT CIR were able to destroy GIST cells. It was demonstrated that 2^{nd} gen designer T cells effectively lysed KIT+ tumor and were more effective than the 1^{st} gen format by LDH release (Figure 4A). To confirm these findings, CFSE-labeled irradiated tumor cells were mixed with unlabeled designer T cells. Tumor cell loss was measured using by quantifying the decrease in CFSE fluorescence from remaining live cells. When compared to CTRL cells, 1^{st} gen and 2^{nd} gen designer T cells mediated significant decreases in the level of CFSE fluorescence and hence number of live tumor cells (Figures 4B-4C). Having demonstrated that the anti-KIT designer T cells were stimulated to divide in vitro in response to KIT+ tumor and lyse KIT+ targets, in vivo efficacy was measured next.

### Example 4: In vivo assessment of anti-KIT designer T cells

To determine the ability of anti-KIT designer T cells to traffic to, infiltrate, and limit growth of established tumor, a subcutaneous xenograft model was utilized. Human KIT+ GIST cells were injected subcutaneously into immunodeficient mice that were treated with tail vein injections of 1^{st} gen, 2^{nd} gen, or unmodified anti-KIT designer T cells seven days later. Tumor measurements were performed in two dimensions (mm²) and are expressed as median percentage change relative to the tumor size on initial day of treatment. IL2 therapy was given along with designer T cells for some groups. Significant reductions in tumor growth were mediated by 1^{st} gen designer T cells without IL2 (p=0.05) and 2^{nd} gen designer T cells (p<0.001) with IL2 (Figure 5A). When all data were pooled, both 1^{st} and 2^{nd} gen designer T cells had a significant impact on tumor growth in the absence of IL2 therapy. With IL2 support, 1^{st} gen designer T cells had a significant effect (p=0.05) while 2^{nd} gen designer T cells (p=0.13) demonstrated a favorable trend (Figure 5B 1^{st} gen dTc may be more reliant on IL2 than 2^{nd} gen dTc because the presence of the co-stimulatory signal through the CD28 portion of the construct may reduce the dependence of 2^{nd} gen dTc on cytokines such as IL2. Data is further represented as tumor growth for each individual sample to demonstrate the range of values (Figure 5C). Following sacrifice, we harvested the tumors and confirmed the presence of adoptively transferred dTc and necrosis in animals treated with 1^{st} or 2^{nd} gen dTc (Figures 5D and 5E).

## Claims

1. A nucleic acid construct for use in a method of treating gastrointestinal stromal tumor (GIST) wherein said GIST is resistant to imatinib mesylate, wherein the nucleic acid construct encodes a chimeric immune receptor CIR protein comprising
a. an extracellular domain of KIT-ligand (KL), or a fragment thereof, wherein said KIT-ligand comprises an amino acid sequence having at least 95% identity to SEQ ID NO:1,
b. a transmembrane domain, and
c. a cytoplasmic domain,
wherein said transmembrane domain comprises a transmembrane domain of CD28, and said cytoplasmic domain comprises a cytoplasmic domain of CD28 and a cytoplasmic domain of the CD3 zeta chain, and
wherein a cell expressing said CIR protein can lyse KIT+ tumor cells *in vitro.*

2. The nucleic acid construct for use according to claim 1, wherein said extracellular domain further comprises an extracellular domain of CD28.

3. A nucleic acid construct for use in a method of treating gastrointestinal stromal tumor (GIST) wherein said GIST is resistant to imatinib mesylate, wherein the nucleic acid construct encodes a chimeric immune receptor (CIR) protein comprising
a. an extracellular domain of KIT-ligand (KL), or a fragment thereof, wherein said KIT-ligand comprises an amino acid sequence having at least 95% identity to SEQ ID NO:1,
b. a transmembrane domain, and
c. a cytoplasmic domain,
wherein said transmembrane domain comprises a transmembrane domain of the CD3 zeta chain, and said cytoplasmic domain comprises a cytoplasmic domain of the CD3 zeta chain, and
wherein a cell expressing said CIR protein can lyse KIT+ tumor cells *in vitro.*

4. The nucleic acid construct for use according to any of the preceding claims, where said KIT-ligand comprises SEQ ID NO:1.

5. The nucleic acid construct for use according to any of claims 1-4, wherein said CIR protein, when expressed in a T cell, is capable of activating said T cell in the presence of a tyrosine-protein kinase KIT+ (KIT+) tumor cell.

6. The nucleic acid construct for use according to any of claims 1-5, wherein said extracellular domain of said CIR protein is capable of interacting with KIT on the surface of a tumor cell when expressed in a T cell.

7. A vector comprising the nucleic acid construct of any of the preceding claims for use in a method of treating gastrointestinal stromal tumor (GIST) wherein said GIST is resistant to imatinib mesylate.

8. A host cell comprising the nucleic acid construct of any of claims 1-6 or the vector of claim 7 for use in a method of treating gastrointestinal stromal tumor (GIST) wherein said GIST is resistant to imatinib mesylate.

9. The host cell for use of claim 8, wherein said host cell is selected from the group consisting of a T cell, a hematopoietic stem cell, a natural killer cell, a natural killer T cell, a B cell, and a cell of monocytic lineage.

10. The host cell for use according to claims 8 or 9, wherein said host cell is autologous to said subject.

11. The host cell for use according to claims 8 or 9, wherein said host cell is not autologous to said subject.

12. The nucleic acid construct or vector or host cell for use according to any of claims 1-11, further comprising administering a second agent.

13. The nucleic acid construct or vector or host cell for use according to claim 12, wherein said second agent is a tyrosine-kinase inhibitor.

14. The nucleic acid construct or vector or host cell for use according to claim 13, wherein said tyrosine-kinase inhibitor is imatinib.

## Patentansprüche

1. Nukleinsäurekonstrukt zur Verwendung in einem Verfahren zur Behandlung von gastrointestinalen Stromatumoren (GIST), wobei der GIST resistent gegen Imatinibmesylat ist, wobei das Nukleinsäurekonstrukt für ein chimäres Immunrezeptor CIR-Protein kodiert, welches
a. eine extrazelluläre Domäne eines KIT-Liganden (KL) oder eines Fragments davon, wobei der KIT-Ligand eine Aminosäuresequenz mit mindestens 95% Identität zu SEQ ID NO:1 umfasst,
b. eine Transmembrandomäne und
c. eine zytoplasmatische Domäne
umfasst,
wobei die Transmembrandomäne eine Transmembrandomäne von CD28 umfasst, und die zytoplasmatische Domäne eine zytoplasmatische Domäne von CD28 und eine zytoplasmatische Domäne der CD3-Zetakette umfasst, und
wobei eine Zelle, welche das CIR-Protein exprimiert, KIT+-Tumorzellen *in vitro* lysieren kann.

2. Nukleinsäurekonstrukt zur Verwendung nach Anspruch 1, wobei die extrazelluläre Domäne ferner eine extrazelluläre Domäne von CD28 umfasst.

3. Nukleinsäurekonstrukt zur Verwendung in einem Verfahren zur Behandlung von gastrointestinalen Stromatumoren (GIST), wobei der GIST resistent gegen Imatinibmesylat ist, wobei das Nukleinsäurekonstrukt für ein chimäres Immunrezeptor (CIR)-Protein kodiert, welches
a. eine extrazelluläre Domäne des KIT-Liganden (KL) oder eines Fragments davon, wobei der KIT-Ligand eine Aminosäuresequenz mit mindestens 95% Identität zu SEQ ID NO:1 umfasst,
b. eine Transmembrandomäne und
c. eine zytoplasmatische Domäne
umfasst,
wobei die Transmembrandomäne eine Transmembrandomäne der CD3-Zetakette und die zytoplasmatische Domäne eine zytoplasmatische Domäne der CD3-Zetakette umfasst, und wobei eine Zelle, welche das CIR-Protein exprimiert, KIT+-Tumorzellen *in vitro* lysieren kann.

4. Nukleinsäurekonstrukt zur Verwendung nach einem der vorhergehenden Ansprüche, wobei der KIT-Ligand SEQ ID NO:1 umfasst.

5. Nukleinsäurekonstrukt zur Verwendung nach einem der Ansprüche 1-4, wobei das CIR-Protein, wenn es in einer T-Zelle exprimiert wird, in der Lage ist, die T-Zelle in Gegenwart einer Tyrosin-Protein-Kinase KIT+ (KIT+) Tumorzelle zu aktivieren.

6. Nukleinsäurekonstrukt zur Verwendung nach einem der Ansprüche 1-5, wobei die extrazelluläre Domäne des CIR-Proteins in der Lage ist, mit KIT auf der Oberfläche einer Tumorzelle zu interagieren, wenn sie in einer T-Zelle exprimiert wird.

7. Vektor, welcher das Nukleinsäurekonstrukt eines der vorhergehenden Ansprüche umfasst, zur Verwendung in einem Verfahren zur Behandlung von gastrointestinalen Stromatumoren (GIST), wobei der GIST resistent gegen Imatinibmesylat ist.

8. Wirtszelle, welche das Nukleinsäurekonstrukt nach einem der Ansprüche 1-6 oder den Vektor nach Anspruch 7 umfasst, zur Verwendung in einem Verfahren zur Behandlung von gastrointestinalen Stromatumoren (GIST), wobei der GIST resistent gegen Imatinibmesylat ist.

9. Wirtszelle zur Verwendung nach Anspruch 8, wobei die Wirtszelle ausgewählt ist aus einer Gruppe bestehend aus einer T-Zelle, einer hämatopoetischen Stammzelle, einer natürlichen Killerzelle, einer natürlichen Killer-T-Zelle, einer B-Zelle und einer Zelle monozytärer Herkunft.

10. Wirtszelle zur Verwendung nach den Ansprüchen 8 oder 9, wobei die Wirtszelle dem Patienten autolog ist.

11. Wirtszelle zur Verwendung nach den Ansprüchen 8 oder 9, wobei die Wirtszelle dem Patienten nicht autolog ist.

12. Nukleinsäurekonstrukt oder Vektor oder Wirtszelle zur Verwendung nach einem der Ansprüche 1-11, ferner umfassend die Verabreichung eines zweiten Agens.

13. Nukleinsäurekonstrukt oder Vektor oder Wirtszelle zur Verwendung nach Anspruch 12, wobei das zweite Agens ein Tyrosin-Kinase-Inhibitor ist.

14. Nukleinsäurekonstrukt oder Vektor oder Wirtszelle zur Verwendung nach Anspruch 13, wobei der Tyrosin-Kinase-Inhibitor Imatinib ist.

## Revendications

1. Construction d'acide nucléique pour utilisation dans un procédé de traitement d'une tumeur stromale gastro-intestinale (TSGI), ladite TSGI étant résistante au mésylate d'imatinib, la construction d'acide nucléique codant pour une protéine de récepteur immunitaire chimérique CIR comprenant
a. un domaine extracellulaire de KIT-ligand (KL), ou un fragment de celui-ci, ledit KIT-ligand comprenant une séquence d'acides aminés ayant au moins 95 % d'identité avec SEQ ID NO: 1,
b. un domaine transmembranaire, et
c. un domaine cytoplasmique,
ledit domaine transmembranaire comprenant un domaine transmembranaire de CD28, et ledit domaine cytoplasmique comprenant un domaine cytoplasmique de CD28 et un domaine cytoplasmique de la chaîne zêta de CD3, et
une cellule exprimant ladite protéine CIR pouvant lyser des cellules tumorales KIT+ *in vitro.*

2. Construction d'acide nucléique pour utilisation selon la revendication 1, dans laquelle ledit domaine extracellulaire comprend en outre un domaine extracellulaire de CD28.

3. Construction d'acide nucléique pour utilisation dans un procédé de traitement d'une tumeur stromale gastro-intestinale (TSGI), ladite TSGI étant résistante au mésylate d'imatinib, la construction d'acide nucléique codant pour une protéine de récepteur immunitaire chimérique (CIR) comprenant
a. un domaine extracellulaire de KIT-ligand (KL), ou un fragment de celui-ci, ledit KIT-ligand comprenant une séquence d'acides aminés ayant au moins 95 % d'identité avec SEQ ID NO: 1,
b. un domaine transmembranaire, et
c. un domaine cytoplasmique,
ledit domaine transmembranaire comprenant un domaine transmembranaire de la chaîne zêta de CD3, et ledit domaine cytoplasmique comprenant un domaine cytoplasmique de la chaîne zêta de CD3, et
une cellule exprimant ladite protéine CIR pouvant lyser des cellules tumorales KIT+ *in vitro.*

4. Construction d'acide nucléique pour utilisation selon l'une quelconque des revendications précédentes, ledit KIT-ligand comprenant SEQ ID NO: 1.

5. Construction d'acide nucléique pour utilisation selon l'une quelconque des revendications 1 à 4, dans laquelle ladite protéine CIR, lorsqu'elle est exprimée dans un lymphocyte T, est capable d'activer ledit lymphocyte T en présence d'une cellule tumorale tyrosine protéine kinase KIT+ (KIT+).

6. Construction d'acide nucléique pour utilisation selon l'une quelconque des revendications 1 à 5, dans laquelle ledit domaine extracellulaire de ladite protéine CIR est capable d'interagir avec KIT sur la surface d'une cellule tumorale lorsqu'elle est exprimée dans un lymphocyte T.

7. Vecteur comprenant la construction d'acide nucléique selon l'une quelconque des revendications précédentes pour utilisation dans un procédé de traitement d'une tumeur stromale gastro-intestinale (TSGI), ladite TSGI étant résistante au mésylate d'imatinib.

8. Cellule hôte comprenant la construction d'acide nucléique selon l'une quelconque des revendications 1 à 6 ou le vecteur selon la revendication 7 pour utilisation dans un procédé de traitement d'une tumeur stromale gastro-intestinale (TSGI), ladite TSGI étant résistante au mésylate d'imatinib.

9. Cellule hôte pour utilisation selon la revendication 8, ladite cellule hôte étant choisie dans le groupe constitué d'un lymphocyte T, d'une cellule souche hématopoïétique, d'une cellule tueuse naturelle, d'un lymphocyte NKT, d'un lymphocyte B, et d'une cellule de lignage monocytaire.

10. Cellule hôte pour utilisation selon les revendications 8 ou 9, ladite cellule hôte étant autologue pour ledit sujet.

11. Cellule hôte pour utilisation selon les revendications 8 ou 9, ladite cellule hôte n'étant pas autologue pour ledit sujet.

12. Construction d'acide nucléique ou vecteur ou cellule hôte pour utilisation selon l'une quelconque des revendications 1 à 11, comprenant en outre l'administration d'un deuxième agent.

13. Construction d'acide nucléique ou vecteur ou cellule hôte pour utilisation selon la revendication 12, ledit deuxième agent étant un inhibiteur de tyrosine kinase.

14. Construction d'acide nucléique ou vecteur ou cellule hôte pour utilisation selon la revendication 13, ledit inhibiteur de tyrosine kinase étant l'imatinib.
